# EUROPEAN PATENT SPECIFICATION

(11) **EP 1 582 547 B1**
(45) Date of publication and mention of the grant of the patent: **25.07.2007**
(21) Application number: 05104685.2
(22) Date of filing: 03.11.2000
(51) Int. Cl.: C08G 63/64, C08G 63/08, A61L 31/06, A61L 17/12

(54) **Vascular graft**
Gefässimplantat
Implant vasculaire

(30) Priority: 30.11.1999 US 167998 P; 27.10.2000 US 698527
(43) Date of publication of application: 05.10.2005
(62) Divisional of application: 00981983.0
(73) Proprietor: Poly-Med, Inc., Anderson, SC 29625 (US)
(72) Inventor: Shalaby, S.W., Dr., ANDERSON, SC 29625 (US)
(74) Representative: Ruff, Michael

(56) References cited:
- EP-A- 0 628 587
- EP-A- 0 774 265
- WO-A-98/00065
- US-A- 5 578 662
- US-A- 5 854 383
- US-A- 6 048 947

## Description

### Background of the Invention

Since the successful development of crystalline thermoplastic polyglycolide as an absorbable fiber-forming material, there has been a great deal of effort directed to the development of new linear fiber-forming polyesters with modulated mechanical properties and absorption profiles. Such modulation was made possible through the application of the concept of chain segmentation or block formation, where linear macromolecular chains comprise different chemical entities with a wide range of physicochemical properties, among which is the ability to crystallize or impart internal plasticization. Typical examples illustrating the use of this strategy are found in U.S. Patent Nos. 5,554,170, 5,431,679, 5,403,347, 5,236,444, and 5,133,739, where difunctional initiators were used to produce linear crystallizable copolymeric chains having different microstructures.

On the other hand, controlled branching in crystalline, homochain polymers, such as polyethylene, has been used as a strategy to broaden the distribution in crystallite size, lower the overall degree in crystallinity and increase compliance (L. Mandelkern, Crystallization of Polymers, McGraw-Hill Book Company, NY, 1964, p. 105-106). A similar but more difficult-to-implement approach to achieving such an effect on crystallinity as alluded to above has been used specifically in the production of linear segmented and block heterochain copolymers such as (1) non-absorbable polyether-esters of polybutylene terephthalate and polytetramethylene oxide [see S. W. Shalaby and H. E. Bair, Chapter 4 of Thermal Characterization of Polymeric Materials (E.A. Turi, Ed.) Academic Press, NY, 1981, p. 402; S. W. Shalaby et al., U.S. Patent 4,543,952 (1985)]; (2) block/segmented absorbable copolymers of high melting crystallizable polyesters such as polyglycolide with amorphous polyether-ester such as poly-1,5-dioxepane-2-one (see A. Kafrawy et al., U.S. Patent 4,470,416 (1984)); and (3) block/segmented absorbable copolyesters of crystallizable and non-crystallizable components as cited in U.S. Patent Nos. 5,554,170, 5,431,679, 5,403,347, 5,236,444, and 5,133,739. However, the use of a combination of controlled branching (polyaxial chain geometry) and chain segmentation or block formation of the individual branches to produce absorbable polymers with tailored properties cannot be found in the prior art. This and recognized needs for absorbable polymers having unique combinations of crystallinity and high compliance that can be melt-processed into high strength fibers and films with relatively brief absorption profiles as compared to their homopolymeric crystalline analogs provided an incentive to explore a novel approach to the design of macromolecular chains to fulfill such needs. Meanwhile, initiation of ring-opening polymerization with organic compounds having three or four functional groups have been used as a means to produce crosslinked elastomeric absorbable systems as in the examples and claims of U.S. Patent No. 5,644,002. Contrary to this prior art and in concert with the recognized needs for novel crystallizable, melt-processable materials, the present invention deals with the synthesis and use of polyaxial initiators with three or more functional groups to produce crystallizable materials with melting temperatures above 100°C, which can be melt-processed into highly compliant absorbable films and fibers.

### Summary of the Invention

The present invention relates to an absorbable, crystalline, monocentric, polyaxial copolymer and a process of preparing the copolymer according to the independent claims. Preferred embodiments are set forth in the dependent claims.

In one aspect the present invention is directed to an absorbable, crystalline, monocentric, polyaxial copolymer which includes a central atom which is carbon or nitrogen and at least three axes originating and extending outwardly from the central atom, with axis including an amorphous, flexible component adjacent to and originating from the central atom, the amorphous component being formed of repeat units derived from at least one cyclic monomer, either a carbonate or a lactone, and a rigid, crystallizable component extending outwardly from the amorphous, flexible component, the crystallizable component being formed of repeat units derived from at least one lactone.

In another aspect the present invention is directed to an absorbable, monocentric, polyaxial copolymer made by a process which includes the steps of:
a) providing a monomeric initiator which is an organic compound selected from the group consisting of tri-functional organic compounds and tetrafunctional organic compounds;
b) providing a catalyst based on a multivalent metal;
c) reacting at least one cyclic comonomer selected from the group consisting essentially of carbonates and lactones with the monomeric initiator in the presence of the catalyst such that an amorphous polymeric, polyaxial initiator is formed by ring-opening polymerization of the at least one cyclic comonomer; and
d) reacting the amorphous, polymeric polyaxial initiator with at least one lactone comprising a member selected from the group consisting of glycolide, lactide, ρ-dioxanone, and combinations thereof.

According to still another aspect of the present invention the subject copolymer is converted to different forms of absorbable stents, to a suture or to a sealing device for closing a wound in a wall of a blood vessel.

### BRIEF DESCRIPTION OF THE DRAWINGS

The foregoing and other aspects of the present invention will be best appreciated with reference to the following detailed description of specific embodiments of the invention, given by way of example only, when read in conjunction with the accompanying drawing, wherein
Figure 1 shows a sealing device for closing a wound in a wall of a vessel according to a first embodiment of a first specific application of the invention.
Figure 2 shows a sectional view of a first sealing member.
Figure 3 shows a sectional view of a second sealing member.
Figure 4 shows a sealing device for closing a wound in a wall of a vessel according to a first embodiment of a first specific application of the invention,
Figure 5 shows an elongated core.
Figure 6 shows a sealing device for closing a wound in a wall of a vessel according to a second embodiment of a first specific application of the invention.
Figure 7 shows a sealing device for closing a wound in a wall of a vessel according to a third embodiment of a first specific application of the invention.
Figure 8 shows a sealing device for closing a wound in a wall of a vessel according to a fifth embodiment of a first specific application of the invention.
Figure 9 shows a sealing device for closing a wound in a wall of a vessel according to a fifth embodiment of a first specific application of the invention.
Figure 10 shows schematically a prior art stent applicable in the present invention.
Figure 11 is a longitudinal view of a stent according to a preferred embodiment of the present invention.
Figure 12 is a cross sectional view of the stent shown in figure 11.
Figure 13 is a longitudinal view of a stent according to another preferred embodiment of the present invention.

### Description of Preferred Embodiments

This invention deals with absorbable, polyaxial, monocentric, crystallizable, polymeric molecules with non-crystallizable, flexible components of the chain at the core and rigid, crystallizable segments at the chain terminals. More specifically, the present invention is directed to the design of amorphous polymeric polyaxial initiators with branches originating from one polyfunctional organic compound so as to extend along more than two coordinates and their copolymerization with cyclic monomers to produce compliant, crystalline film- and fiber-forming absorbable materials. The absorbable copolymeric materials of this invention comprise at least 30 percent, and preferably 65 percent, by weight, of a crystallizable component which is made primarily of glycolide-derived or 1-lactide-derived sequences, and exhibit first and second order transitions below 222°C and below 42°C, respectively, and undergo complete dissociation into water-soluble by-products in less than 180 days and preferably 120 days when incubated in a phosphate buffer at 37°C and pH 7.4 or implanted in living tissues.

The amorphous polymeric, polyaxial initiators (PPIs) used in this invention to produce crystalline absorbable copolymeric materials can be made by reacting a cyclic monomer or a mixture of cyclic monomers such as trimethylene carbonate, ε-caprolactone, and 1,5-dioxapane-2-one in the presence of an organometallic catalyst with one or more polyhydroxy, polyamino, or hydroxyamino compound having more than three reactive amines and/or hydroxyl groups. Typical examples of the latter compounds are glycerol, ethane-trimethylol, propane-trimethylol, pentaerythritol, a partially alkylated cyclodextrin, triethanolamine, N-2-aminoethyl-1,3-propanediamine, 3-amino-5-hydroxy pyrazole, and 4-amino-6-ydroxy-2-mercapto-pyrimidine.

The crystalline copolymers of the present invention are so designed to (1) have the PPI devoid of any discernable level of crystallinity; (2) have the PPI component function as a flexible spacer of a terminally placed, rigid, crystallizable component derived primarily from glycolide so as to allow for facile molecular entanglement to create pseudo-crosslinks, which in turn, maximize the interfacing of the amorphous and crystalline fractions of the copolymer leading to high compliance without compromising tensile strength; (3) maximize the incorporation of the hydrolytically labile glycolate linkage in the copolymer without compromising the sought high compliance-this is achieved by directing the polyglycolide segments to grow on multiple active sites of the polymeric initiator and thus limiting the length of the crystallizable chain segments; (4) have a broad crystallization window featuring maximum nucleation sites and slow crystallite growth that in turn assists in securing a highly controlled post-processing and development of mechanical properties-this is achieved by allowing the crystallizable components to entangle effectively with non-crystallizable components leading to high affinity for nucleation, high precrystallization viscosity, slow chain motion, and low rate of crystallization; (5) force the polymer to form less perfect crystallites with broad size distribution and lower their melting temperature as compared to their homopolymeric crystalline analogs to aid melt-processing-this is achieved by limiting the length of the crystallizable segments of the copolymeric chain as discussed earlier; (6) allow for incorporating basic moieties in the PPI which can affect autocatalytic hydrolysis of the entire system which in turn accelerates the absorption rate; and (7) allow the polymer chain to associate so as to allow for endothermic thermal events that can be related to tensile toughness similar to that detected in PET relative to the so-called middle endothermic peak (MEP) (S. W. Shalaby, Chapter 3 of Thermal Characterization of Polymeric Materials, Academic press, NY, 1981, p. 330).

As an example, the crystalline copolymeric materials of the present invention may be prepared as follows, although as noted above, other monomers are also within the scope of the present invention. The amorphous polymeric polyaxial initiator is formed by a preliminary polymerization of a mixture of ε-caprolactone and trimethylene carbonate in the presence of trimethylol-propane and a catalytic amount of stannous octoate, using standard ring-opening polymerization conditions which entail heating the stirred reactants in nitrogen atmosphere at a temperature exceeding 110°C until substantial or complete conversion of the monomers is realized. This can be followed by adding a predetermined amount of glycolide. Following the dissolution of the glycolide in the reaction mixture, the temperature is raised above 150°C to allow the glycolide to copolymerize with the polyaxial initiator. When practically all the glycolide is allowed to react, the resulting copolymer is cooled to 25°C. After removing the polymer from the reaction kettle and grinding, trace amounts ofunreacted monomer are removed by heating under reduced pressure. The ground polymer can then be extruded and pelletized prior to its conversion to fibers or films by conventional melt-processing methods. At the appropriate stage of polymerization and product purification, traditional analytical methods, such as gel-permeation chromatography (GPC), solution viscosity, differential scanning calorimetry (DSC), nuclear magnetic resonance (NMR), and infrared spectroscopy (IR) are used to monitor or determine (directly or indirectly) the extent of monomer conversion, molecular weight, thermal transitions (melting temperature, Tₘ, and glass transition temperature, T_{g}), chain microstructure, and chemical entity, respectively.

Another aspect of this invention deals with end-grafting a PPI with ε-caprolactone or 1-lactide, and preferably in the presence of a minor amount of a second monomer, to produce absorbable crystalline polymers for use as bone sealants or barrier membranes, respectively.

Films made by compression molding of the copolymers described in the examples set forth below are evaluated for (1) tensile strength; (2) *in vitro* breaking strength retention and mass loss during incubation in a phosphate buffer at 37°C and pH 7.4; (3) *in vivo* breaking strength retention using a rat model where strips of the films are implanted subcutaneously for 1 to 6 weeks and individual lengths are explanted periodically to determine percent of retained breaking strength; and (4) *in vivo* absorption (in terms of mass loss) using a rat model where a film strip, inserted in a sealed polyethylene terephthalate (PET) woven bag, is placed in the peritoneum for 6, 8, 10, 12 and 14 weeks. At the end of each period, the PET bag is removed and the residual mass of the strips is removed, rinsed with water, dried, and its weight is determined.

Specifically, an important aspect of this invention is the production of compliant absorbable films with modulated absorption and strength loss profiles to allow their use in a wide range of applications as vascular devices or components therefor. More specifically is the use of these devices in sealing punctured blood vessels.

In another aspect, this invention is directed to the use of the polymers described herein for the production of extruded or molded films for use in barrier systems to prevent post-surgical adhesion or compliant covers, sealants, or barriers for burns and ulcers as well as compromised/damaged tissue. The aforementioned articles may also contain one or more bioactive agent to augment or accelerate their functions. In another aspect, this invention is directed to melt-processed films for use to patch mechanically compromised blood vessels. In another aspect, this invention is directed to the use of the polymer described herein as a coating for intravascular devices such as catheters and stents. In another aspect, this invention is directed to the application of the polymers described herein in the production of extruded catheters for use as transient conduits and microcellular foams with continuous porous structure for use in tissue engineering and guiding the growth of blood vessels and nerve ends. Another aspect of this invention is directed to the use of the polymers described herein to produce injection molded articles for use as barriers, or plugs, to aid the function of certain biomedical devices used in soft and hard tissues and which can be employed in repairing, augmenting, substituting or redirecting/assisting the functions of several types of tissues including bone, cartilage, and lung as well as vascular tissues and components of the gastrointestinal and urinogenital systems. In another aspect, this invention is directed to the use of polymers described herein to produce compliant, melt-blown fabrics and monofilament sutures with modulated absorption and strength retention profiles.

In one aspect of this invention, the subject copolymers are converted to different forms of absorbable stents, such as those used (1) as an intraluminal device for sutureless gastrointestinal sutureless anastomosis; (2) in laparoscopic replacement of urinary tract segments; (3) as an intraluminal device for artery welding; (4) in the treatment of urethral lesions; (5) as a tracheal airway; (6) in the treatment of recurrent urethral strictures; (7) for vasectomy reversal; (8) in the treatment of tracheal stenoses in children; (9) for vasovasostomy; (10) for end-to-end ureterostomy; and (11) as biliary devices.

In another aspect of this invention, the subject copolymers are converted to a highly compliant, expandable tubular mantle, sleeve or cover that is placed tightly outside an expandable metallic or polymeric stent so that under concentric irreversible expansion at the desired site of a treated biological conduit, such as blood vessel or a urethra, both components will simultaneously expand and the mantle provides a barrier between the inner wall of the conduit and the outer wall of the stent. In another aspect of this invention, the subject copolymers are used as a stretchable matrix of a fiber-reinforced cover, sleeve, or mantle for a stent, wherein the fiber reinforcement is in the form of spirally coiled yarn (with and without crimping) woven, knitted, or braided construct. In another aspect of this invention, the stent mantle, or cover, is designed to serve a controlled release matrix of bioactive agents such as those used (1) for inhibiting neointima formation as exemplified by hirudin and the prostacyclic analogue, iloprost; (2) for inhibiting platelet aggregation and thrombosis; (3) for reducing intraluminal and particular intravascular inflammation as exemplified by dexamethasone and non-steroidal inflammatory drugs, such as naproxen; and (4) for suppressing the restenosis.

One aspect of this invention deals with the conversion of the subject copolymers into molded devices or components of devices used as a hemostatic puncture closure device after coronary angioplasty.

It is further within the scope of this invention to incorporate one or more medico-surgically useful substances into the copolymers and devices subject of this invention. Typical examples of these substances are those capable of (1) minimizing or preventing platelet adhesion to the surface of vascular grafts; (2) rendering anti-inflammatory functions; (3) blocking incidents leading to hyperplasia as in the case of synthetic vascular grafts; (4) aiding endothelialization of synthetic vascular grafts; (5) preventing smooth muscle cell migration to the lumen of synthetic vascular grafts; and (6) accelerating guided tissue ingrowth in fully or partially absorbable scaffolds used in vascular tissue engineering.

In order that those skilled in the art may be better able to practice the present invention, the following illustrations of the preparation of typical crystalline copolymers are provided.

### EXAMPLE 1: Synthesis of 20/25 (molar) ε-Caprolactone/Trimethylene Carbonate Copolymer as a Tri-axial Initiator and Reaction with 55 Relative Molar Parts of Glycolide

An initial charge consisted of 142.4 grams (1.249 moles) ε-caprolactone, 159.4 grams (1.563 moles) trimethylene carbonate, 1.666 grams (1.24×10⁻² moles) trimethylol-propane, and 1.0 ml (2.03×10⁻⁴ moles) of a 0.203M solution of stannous octoate catalyst in toluene after flame drying the reaction apparatus. The reaction apparatus was a 1 L stainless steel kettle with 3-neck glass lid equipped with an overhead mechanical stirring unit, vacuum adapter, and two 90° connectors for an argon inlet.

The apparatus and its contents were heated to 50°C under vacuum with a high temperature oil bath. Upon complete melting of the contents after 30 minutes, the system was purged with argon, stirring initiated at 32 rpm, and the temperature set to 150°C. After 4 hours at 150°C, the viscosity of the polyaxial polymeric initiator (PPI) had increased and the temperature of the bath was reduced to 110°C. Upon reaching 110°C, 398.5 grams (3.435 moles) of glycolide were added to the system. When the glycolide had completely melted and mixed into the polyaxial polymeric initiator, the temperature was increased to 180°C and stirring was stopped. The reaction was allowed to continue for 2 hours before cooling the system to 50°C and maintaining the heat overnight. The polymer was isolated, ground, dried, extruded and redried as described below in Example 5.

The extrudate was characterized as follows: The inherent viscosity using hexafluoroisopropyl alcohol (HFIP) as a solvent was 0.97 dL/g. The melting temperature and heat of fusion, as determined by differential scanning calorimetry (using initial heating thermogram), were 215°C and 40.8 J/g, respectively.

### EXAMPLE 2: Synthesis of 25/30 (molar) ε-Caprolactone/Trimethylene Carbonate Copolymer as a Tri-axial Initiator and Reaction with 45 Relative Molar Parts of Glycolide

An initial charge consisted of 122.8 grams (1.077 moles) ε-caprolactone, 131.9 grams (1.292 moles) trimethylene carbonate, 1.928 grams (1.44×10⁻² moles) trimethylol-propane, and 1.0 ml (8.62×10⁻⁵ moles) of a 0.086M solution of stannous octoate catalyst in toluene after flame drying the reaction apparatus. The reaction apparatus was a 1 L stainless steel kettle with 3-neck glass lid equipped with an overhead mechanical stirring unit, vacuum adapter, and two 90° connectors for an argon inlet.

The apparatus and its contents were then heated to 65°C under vacuum with a high temperature oil bath. After 30 minutes, with the contents completely melted, the system was purged with argon, stirring initiated at 34 rpm, and the temperature set to 140°C. After 3 hours at 140°C, the temperature was raised to 150°C for 1 hour and then reduced back to 140°C. At this point, 225.0 grams (1.940 moles) of glycolide were added to the system while rapidly stirring. When the glycolide had completely melted and mixed into the polyaxial polymeric initiator, the temperature was increased to 180°C and stirring was stopped. The reaction was allowed to continue for 2 hours before cooling the system to room temperature overnight. The polymer was isolated, ground, dried, extruded, and redried as described in Example 5.

Characterization of the extrudate was conducted as follows: The inherent viscosity using HFIP as a solvent was 0.93 dL/g. The melting temperature and heat of fusion, as measured by differential scanning calorimetry (DSC using initial heating thermogram), were 196°C and 32.1 J/g, respectively.

### EXAMPLE 3: Synthesis of 20/25/3 (molar) ε-Caprolactone/Trimethylene Carbonate/Glycolide Copolymer as a Tri-axial Initiator and Reaction with 52 Relative Molar Parts of Glycolide

An initial charge consisted of 101.6grams (0.891 moles) ε-caprolactone, 113.5 grams (1.113 moles) trimethylene carbonate, 15.5 grams of glycolide (0.134 moles), 1.996 grams (1.49×10⁻² moles) trimethylol-propane, and 1.0 ml (1.28×10⁻⁴ moles) of a 0.128M solution of stannous octoate catalyst in toluene after flame drying the reaction apparatus. The reaction apparatus was a 1 L stainless steel kettle with 3-neck glass lid equipped, an overhead mechanical stirring unit, vacuum adapter, and two 90° connectors for an argon inlet.

The apparatus and its contents were then heated to 85°C under vacuum with a high temperature oil bath. After 30 minutes, with the contents completely melted, the system was purged with argon, stirring initiated at 34 rpm, and the temperature set to 140°C. After 4 hours at 140°C, 268.8 grams (2.317 moles) of glycolide were added to the system while rapidly stirring. When the glycolide had completely melted and mixed into the polyaxial polymeric initiator, the temperature was increased to 180°C and stirring was stopped. The reaction was allowed to continue for 2 hours before cooling the system to room temperature overnight. The polymer was isolated, ground, dried, extruded and redried as in Example 5.

The extrudate was characterized as follows: The inherent viscosity using HFIP as a solvent was .89 dL/g. The melting temperature and heat of fusion, as measured by differential scanning calorimetry (DSC using initial heating thermogram), were 212°C and 34 J/g, respectively.

### EXAMPLE 4: Synthesis of 20/25/3 (molar) ε-Caprolactone/Trimethylene-Carbonate/Glycolide Copolymer as a Tri-axial Initiator and Reaction with 52 Relative Molar Parts of Glycolide

Glycolide (18.6 g, 0.1603 mole), TMC (136.7 g, 1.340 mole), ε-caprolactone (122.0 g, 1.070 mole), trimethylolpropane (2.403 g, .01791 mole) and stannous octoate catalyst (0.2M in toluene, 764 µL, 0.1528 mmol) were added under dry nitrogen conditions to a 1.0 liter stainless steel reaction kettle equipped with a glass top and a mechanical stirrer. The reactants were melted at 85°C and the system was evacuated with vacuum. The system was purged with dry nitrogen and the melt was heated to 160°C with stirring at 30 rpm. Samples of the prepolymer melt were taken periodically and analyzed for monomer content using GPC. Once the monomer content of the melt was found to be negligible, glycolide (322.5 g, 2.780 mole) was added with rapid stirring. The stir rate was lowered to 30 rpm after the contents were well mixed. The melt was heated to 180°C. Stirring was stopped upon solidification of the polymer. The polymer was heated for 2 hours at 180°C after solidification. The resulting polymer was cooled to room temperature, quenched in liquid nitrogen, isolated, and dried under vacuum. The polymer was isolated, ground, redried, and extruded as described in Example 5. The extrudate was characterized by NMR and IR for identity and DSC (using initial heating thermogram) for thermal transition (Tₘ = 208°C, ΔH = 28.0 J/g) and solution viscosity in hexafluoroisopropyl alcohol (η = 0.92 dL/g).

### EXAMPLE 5: Size Reduction and Extrusion of Polymers of Examples 1 through 4

The polymer was quenched with liquid nitrogen and mechanically ground. The ground polymer was dried under vacuum at 25°C for two hours, at 40°C for two hours, and at 80°C for four hours. The polymer was melt extruded at 225°C to 235°C using a ½ inch extruder equipped with a 0.094 in die. The resulting filaments were water cooled. The average filament diameter was 2.4 mm. The filament was dried at 40°C and 80°C under vacuum for eight and four hours, respectively.

### EXAMPLE 6: Compression-molding of Polymers from Examples 3 and 4 to a Sealing Device for a Punctured Blood Vessel and Its Packaging

The compression molding process entailed exposing the polymer to an elevated temperature between two mold halves. When temperature of the mold halves exceeded the polymer melting temperature, pressure was applied to the mold and the material was allowed to flow into a predefined cavity of the mold. The mold was then cooled to room temperature before it was opened and the newly shaped polymer was removed.

The full molding cycle can be described as: (1) Drying-typical: temperature 80°C during 2 hours; (2) Pre-heating, temperature increase-typical: pressure 5,000N, temperature from room temperature up to 200°C; (3) Forming, constant temperature under high pressure-typical: pressure 50,000N, temperature 200°C; (4) Cooling, temperature decrease under high pressure-typical: pressure 50,000N, temperature from 200°C down to 50°C; (5) Mold opening; (6) Annealing-typical: temperature 80°C during 2 hours; and (7) Packaging-typically the device was removed from the mold and packaged under vacuum under a protective gas environment.

### EXAMPLE 7: Synthesis of 13.3/17.7/2 (molar) Caprolactone/Trimethylene Carbonate/Glycolide Copolymer as a Tri-axial Initiator and Reaction with Relative 67 Molar Parts of Glycolide

Glycolide (10.4 g, 0.090 mole), TMC (76.5 g, 0.750 mole), ε-caprolactone (68.4 g, 0.600 mole), trimethylolpropane (1.995 g, .01487 mole) and stannous octoate catalyst (0.2M in toluene, 637 µL, 0.1274 mmole) were added under dry nitrogen conditions to a 1.0 liter stainless steel reaction kettle equipped with a glass top and a mechanical stirrer. The reactants were melted at 85°C and the system was evacuated with vacuum. The system was purged with dry nitrogen and the melt was heated to 160°C with stirring at 30 rpm. Samples of the prepolymer melt were taken periodically and analyzed for monomer content using GPC. Once the monomer content of the melt was found to be negligible, glycolide (344.5 g, 2.970 mole) was added with rapid stirring. The stir rate was lowered to 30 rpm after the contents were well mixed. The melt was heated to 180°C. Stirring was stopped upon solidification of the polymer. The polymer was heated for 2 hours at 180°C after solidification. The resulting polymer was cooled to room temperature, quenched in liquid nitrogen, isolated, and dried under vacuum. The polymer was characterized by NMR and IR (for identity), DSC thermal transition (Tₘ = 215.7) and solution viscosity in hexafluoroisopropyl alcohol (η - 0.95 dL/g).

### EXAMPLE 8: Synthesis of 13.6/17.0/2.0 (molar) ε-Caprolactone/Trimethylene Carbonate/Glycolide Copolymer as a Basic Tri-axial Initiator and Reaction with Relative 67.4 Molar Parts of Glycolide and Trimethylene Carbonate

Glycolide (3.1 g, 0.0267 mole), TMC (23.0 g, 0.2255 mole), ε-caprolactone (20.5 g, 0.1798 mole), triethanolamine (0.6775 g, 4.55 mmole) and stannous octoate catalyst (0.2M in toluene, 519 µL, 0.1038 mmole) were added under dry nitrogen conditions to a 0.5 Liter stainless steel reaction kettle equipped with a glass top and a mechanical stirrer. The reactants were melted at 85°C and the system was evacuated with vacuum. The system was purged with dry nitrogen and the melt was heated to 160°C with stirring at 30 rpm. Samples of the prepolymer melt were taken periodically and analyzed for monomer content using GPC. Once the monomer content of the melt was found to be negligible, glycolide (103.4 g, 0.8914 mole) was added with rapid stirring. The stir rate was lowered to 30 rpm after the contents were well mixed. The melt was heated to 180°C. Stirring was stopped upon solidification of the polymer. The polymer was heated for 2 hours at 180°C after solidification. The resulting polymer was cooled to room temperature, quenched in liquid nitrogen, isolated, and dried under vacuum. The polymer was characterized for identity and composition (IR and NMR, respectively) and thermal transition by DSC (Tₘ 220°C) and molecular weight by solution viscometry (η = 0.80 in hexafluoroisopropyl alcohol).

### EXAMPLE 9: Synthesis of 13.6/17.0/2.0 (molar) ε-Caprolactone/Trimethylene Carbonate/Glycolide Copolymer as a Basic Tri-axial Initiator and Reaction with Relative 67.4 Molar Parts of Glycolide

The two-step polymerization was conducted as in Example 8 with the exception of using 0.6915 g triethanolamine and 693µl of stannous octanoate solution. The final polymer was isolated and characterized as in Example 8 and it was shown to have a Tₘ = 221°C and inherent viscosity (in HFIP) = 0.82.

### EXAMPLE 10: Synthesis of 13.3/17.7/2 (molar) ε-Caprolactone/Trimethylene Carbonate/Glycolide Copolymer as a Tetra-axial Initiator and Reaction with Relative 67 Molar Parts of Glycolide

Glycolide (3.1 g, 0.0267 mole), TMC (23.0g, 0.2255 mole), ε-caprolactone (20.5 g, 0.1796 mole), pentaerythritol (0.600 g., 0.0044 mole) and stannous octoate catalyst (0.2 M in toluene, 193 µl, 0.0386 mmol) were placed under dry nitrogen conditions to a 0.5 L stainless steel reaction kettle equipped with a glass top and a mechanical stirrer. The polymerization charge was dried at 25°C and 40°C under reduced pressure for 60 and 30 minutes, respectively. The reactants were then melted at 85°C and the system was purged with dry nitrogen. The melt was heated to 160°C with stirring at 30 rpm. Samples of the prepolymer melt were taken periodically and analyzed for monomer content using GPC (gel permeation chromatography). Once the monomer content of the polymer melt was found to be negligible, glycolide (103.4 g., 0.8914 mole) was added with rapid stirring that is more than 40 rpm. The stirring rate was then lowered to 30 rpm after the contents were well mixed. The reactants were heated to 180°C. Stirring was stopped upon solidification of the polymer. The polymer was heated for 2 hours at 180°C after solidification. The resulting polymer was cooled to room temperature, quenched in liquid nitrogen, isolated, and dried at 25°C and then 40°C under reduced pressure.

The final polymer was isolated and characterized as in Example 8 and it was shown to have a Tₘ = 219°C and inherent viscosity (in HFIP) = 0.98.

### EXAMPLE 11: Size Reduction and Extrusion of Polymer from Examples 7 through 10

The polymer was quenched with liquid nitrogen and mechanically ground. The ground polymer was dried under vacuum at 25°C for two hours, at 40°C for two hours, and at 80°C for four hours. The polymer was melt extruded at 235°C to 245°C using a ½ inch extruder equipped with a 0.094 in die. The resulting monofilament was quenched in an ice-water bath before winding. The monofilament was dried at 40°C and under vacuum for four hours before orientation.

### EXAMPLE 12: Orientation of Melt-spun Monofilaments

Polymers of Examples 7 through 10 that have been extruded as described in Example 11 were oriented by two-stage drawing into monofilament sutures. Prior to drawing Example 7, monofilaments were pre-tensioned and annealed. The drawing was conducted at 90-100°C in the first stage and 100-130°C in the second stage. The overall draw ratio varied between 3.73X and 4.6X. A number of monofilaments were relaxed at 70°C for 15 minutes to reduce their free shrinkage. Properties of the oriented monofilaments are summarized in Table I.

**Table I. Drawing Conditions and Fiber Properties of Polymers from Examples 7 through 9**

| Origin of Extruded Polymer | Fiber Number | Draw Ratio | Draw Temp. (S1/S2) | Pre-Draw Annealing (min/°C) | Post-Draw Relaxation (%) | Free Shrinkage (%) | Diameter (mil) | Straight Strength (Kpsi) | Modulus (Kpsi) | Etongation (%) |
|---|---|---|---|---|---|---|---|---|---|---|
| Example 7 | 7F-1 | 3.73X | 95/130 | 35/65 | -- | 4.4 | 13.4 | 75 | 444 | 22 |
| 7 | 7F-2 | 3.73X | 95/130 | 35/65 | 2.3 | 1.8 | 15.1 | 53 | 182 | 36 |
| 7 | 7F-3 | 4.14X | 95/120 | 30/65 | -- | 4.2 | 10.2 | 66 | 434 | 19 |
| 7 | 7F-4 | 4.14X | 95/120 | 30/65 | 3 | 1.5 | 11.0 | 61 | 257 | 31 |
| 8 | 8F-1 | 4.50X | 100/120 | -- | -- | 3.1 | 10.2 | 71 | 195 | 26 |
| 9 | 9F-1 | 4.43X | 100/130 | -- | -- | 2.1 | 10.6 | 72 | 230 | 27 |
| 10 | 10F-1 | 4.60X | 95/120 | -- | -- | 2.4 | 12.6 | 57 | 158 | 25 |

### EXAMPLE 13: Sterilization of Monofilament Sutures and Evaluation of Their In Vitro Breaking Strength Retention

Monofilament sutures Numbers 8F-1 and 9F-1 described in Table I were radiochemically sterilized in hermetically sealed foil packages that have been pre-purged with dry nitrogen gas, using 5 and 7.5 KGy of gamma radiation. The radiochemical sterilization process entails the use of 200-400 mg of Delrin (poly-formaldehyde) film as package inserts for the controlled release, radiolytically, of formaldehyde gas as described earlier by Correa et al., [Sixth World Biomaterials Congress, *Trans Soc. Biomat.,* II, 992 (2000)]. The sterile monofilament sutures were incubated in a phosphate buffer at 37°C and pH = 7.4 to determine their breaking strength retention profile as absorbable sutures. Using the breaking strength data of non-sterile sutures (Table I), the breaking strength retention data of sterile sutures were calculated. A summary of these data are given in Table II. These data indicate all sutures retained measurable strength at two weeks in the buffer solution.

**Table II. Tensile Properties and In Vitro Breaking Strength Retention (BSR) of Radiochemically Sterilized Monofilament Sutures**

| Suture Number | 9F-1 | | 8F-1 | |
|---|---|---|---|---|
| Sterilization Dose (KGy) | 5 | 7.5 | 5 | 7.5 |
| Post-irradiation Tensile Properties | | | | |
| • Tensile Strength (Kpsi) | 66 | 68 | 67 | 65 |
| • Modulus (Kpsi) | 266 | 254 | 269 | 263 |
| • Elongation (Kpsi) | 30 | 35 | 31 | 30 |
| BSR, % at | | | | |
| • Week 1 | 70 | 57 | 82 | 72 |
| • Week 2 | 24 | 22 | 18 | 17 |

As indicated above a number of different applications for the copolymer exist. Below two specific applications, namely a device for sealing punctured blood vessels and a stent, will be described more thoroughly.

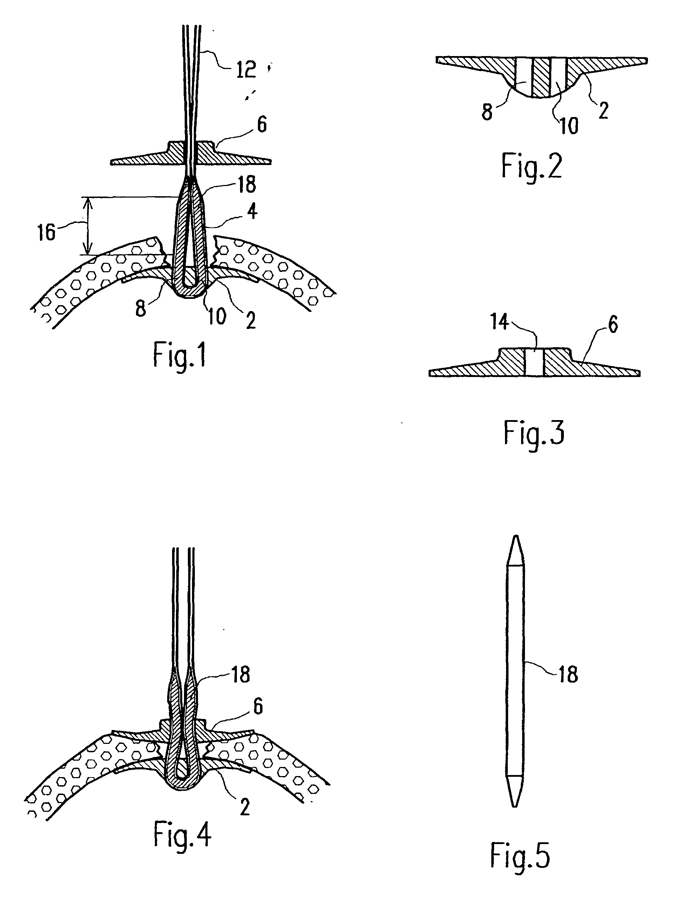

Figure 1 shows a sealing device for closing a wound in a wall of a vessel according to a first embodiment of the invention. The sealing device comprises three separate parts, namely a first sealing member 2 an elongated member 4 and a second sealing member 6. The first sealing member 2 is attached to a distal end of the elongate member 4. In this first embodiment of the sealing device, the first sealing member comprises two through openings 8, 10 (Figure 2) through which a multifilament suture wire 12 is thread so as to make a pair of suture wires constituting the elongated member 4.

The second sealing member 6 is provided with an opening 14 (Figure 3), which is adapted to the elongate member 4, i.e. the opening 14 is greater than the thickness of the proximal portion of the elongate member 4. With a structure like this the second sealing member 6 is threadable onto and along the elongate element 4 (Figure 1). The most distal portion of the elongate member 4 has a constant thickness that is slightly greater than the opening 14 of the second sealing member 6 and constitutes the distal lock portion 16.

This will allow for frictional engagement between inside of the opening 14 of the second sealing member 6 and the distal lock portion 16 of the elongate member 4 which makes the sealing device infinitely variable lockable along said distal lock portion 16 (Figure 4).

The multifilament suture wire 12 is preferably made of a resorbable material such as Glycolic/Lactide polymer The first sealing member 2 and second sealing member 6 are made of the flexible resorbable copolymer according to claims 1-9.

The choice of using a suture wire for the elongated element 4 is very important for the security of the sealing device. Tests have been made to use the same material, e.g. a polymer, in the elongated member 4 as in the second sealing member 6. Since polymer gives a very glossy surface, it is hard to get high power frictional engagement between the elongated member 4 and the sealing member 6. Using a suture wire 12 for the elongate member 4 gives a safer sealing since the suture wire comprises a number of circulating fibres thus giving the wire a rough surface with a high frictional sealing power towards a glossy surface inside the opening 14 of the second sealing member 6.

The suture wire also makes the sealing device safer in another way. The suture wire is made in one piece and has very high tensile strength. It constitutes a continuos wire from the inner seal through the outer seal and to an tampering grip of the insertion tool, being threaded in through the first opening 8 and out again through the second opening 10 and thus keeping the sealing device safe together.

If a first sealing member and an elongated member are cast in one piece there is often problem with the casting process, giving the casted member air bubbles and inclusions and accordingly giving the sealing device poor structural strength.

The challenge is to make the suture wire 12 thicker in the distal lock portion 16.

In the first embodiment of the present invention, a hollow core of the suture wire 12 is filled with an elongated core 18 (Figure 5), within the area of the distal lock portion 16 of the elongate member 4, but also in the area which is to be threaded through the first sealing member 2. (See again Figure 1). The elongated core 18 is preferably made of the resorbable copolymer according to claims 1-9. This gives the suture wire 12 a thickening in the distal lock portion 16.

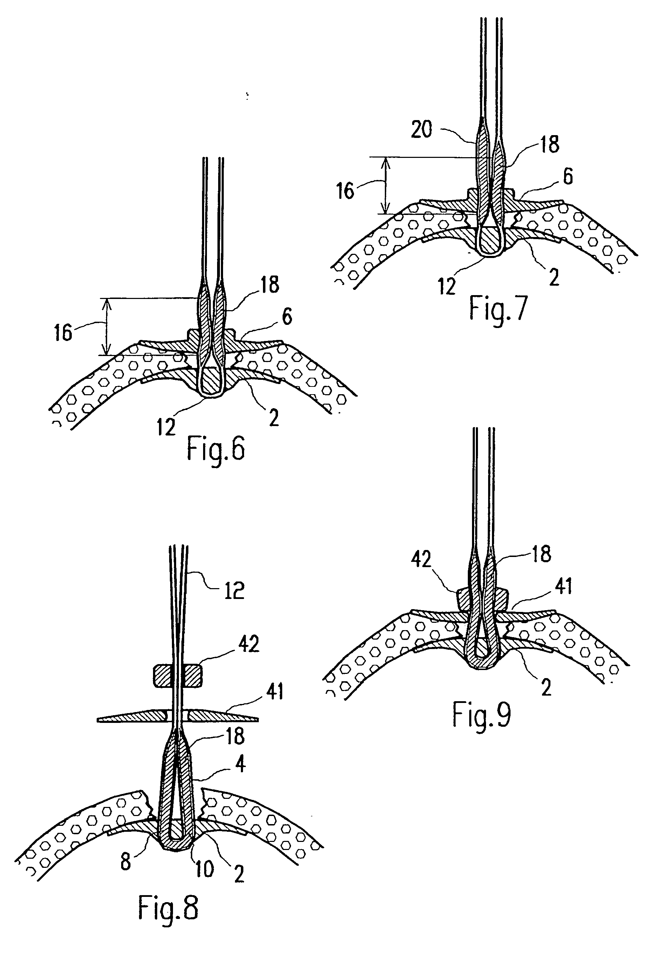

In a second embodiment of the present invention, shown in Figure 6, the suture wire 12 is left unfilled within the area ranging from the entry of the first opening 8 of the first sealing member 2, through the first sealing member 2, out on the on the other side and in again through the second opening 10 of the first sealing member 2 to the exit of said second opening 10.

In a third embodiment of the present invention, shown in Figure 7, the thickening of the first suture, of the two sutures making a pair of sutures, extends beyond the distal lock portion 16 into the proximal portion of the elongated member 4. This gives the suture wire 12 a more continuos increasing of the thickness which simplifies the threading of the second sealing member 6 from the proximal portion onto the distal lock portion 16.

In a forth embodiment of the present invention, instead of being filled, the suture 12 is thicker woven in the area of the distal lock portion.

In a fifth embodiment of the present invention, (Figures 8 and 9) the second sealing member is divided into two parts, which first part 41 is a plate and is provided with an opening that is approximately the same or slightly grater than thickness of the distal lock portion 16. This first part 41 is threadable onto and along the elongate member 4 (Figure 8), over the distal lock portion until it is in contact with the outside of the vessel wall. The first part plate 41 is preferably quite thin, which makes it flexible and easy to adapt to the vessel wall. The second part 42 is provided with an opening that is slightly smaller than the thickness of the distal lock portion 16. This second part 42 is threadable onto and along the elongate member 4 (Figure 8), over the distal lock portion until it is in contact with the first part 41. The second part 42 allows for frictional engagement between the inside of the opening of the second part 42 and the distal portion 16 (Figure 9). The second part 42 is preferably thicker than the first part 41, which will give it a large surface inside its opening for said frictional engagement. On the other hand, the diameter of he second part 42 is preferably smaller than the first part 41.

In a sixth embodiment, the elongated portion 4 is not a suture wire, but another material, e.g. a resorbable polymer. The distal lock portion 16 is coated by a hollow, stocking-like suture wire so that a decent frictional engagement can be achieved between said coated distal lock portion and the inside of the opening of the second sealing member.

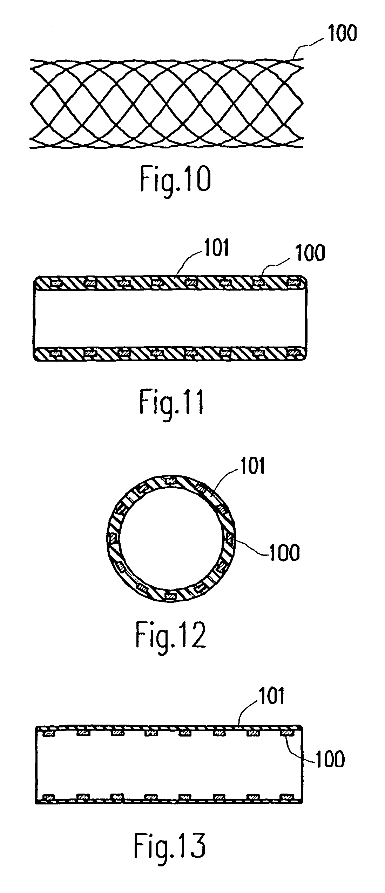

As mentioned above, the subject copolymers may be converted to a highly compliant, expandable tubular mantle, sleeve or cover that is placed tightly outside an expandable metallic or polymeric stent so that under concentric irreversible expansion at the desired site of a treated biological conduit, such as blood vessel or a urethra, both components will simultaneously expand and the mantle provides a barrier between the inner wall of the conduit and the outer wall of the stent. In another aspect of this invention, the subject copolymers are used as a stretchable matrix of a fiber-reinforced cover, sleeve, or mantle for a stent, wherein the fiber reinforcement is in the form of spirally coiled yarn (with and without crimping) woven, knitted, or braided construct. Figure 10 shows schematically a radially expandable prior art spirally coiled metal stent applicable in the present invention.
Figure 11 is a longitudinal view of a stent where the metal stent 100 is completely covered by the subject copolymer 101 according to a preferred embodiment of the present invention.
Figure 12 is a cross sectional view of the stent shown in figure 11.
Figure 13 is a longitudinal view of a stent where the outer surface is covered by the subject copolymer 101 according to another preferred embodiment of the present invention.

The size of a stent depends naturally of the intended use, i.e. the dimensions of the vessel where it should be applied. Typical coronary stent dimensions may have a pre deployment outer diameter of 1.6 mm and an expanded outer diameter of 3.0 mm to 4.5 mm. The length is preferably 15 mm or 28 mm.

Although the present invention has been described in connection with the preferred embodiments, it is to be understood that modifications and variations may be utilized without departing from the principles and scope of the invention, as those skilled in the art will readily understand. Accordingly, such modifications may be practised within the scope of the following claims. Moreover, Applicants hereby disclose all subranges of all ranges disclosed herein. These subranges are also useful in carrying out the present invention.

## Claims

1. A vascular graft comprising an absorbable, crystalline, monocentric, polyaxial copolymer comprising:
a central atom selected from the group consisting of carbon and nitrogen; and at least three axes originating and extending outwardly from the central atom, each axis comprising:
an amorphous, flexible component adjacent to and originating from the central atom, the amorphous component comprising repeat units derived from at least one cyclic monomer selected from the group consisting essentially of carbonates and lactones; and
at least 30 percent, preferably 65 percent, by weight, of a rigid, crystallizable component extending outwardly from the amorphous, flexible component, the crystallizable component being made primarily of glycolide-derived or ℓ-lactide-derived sequences,
wherein glycerol, trimethylolethane, trimethylolpropane or pentaerythritol is used as an initiator in the preparation of the copolymer when the central atom is carbon, and wherein preferably one or more medico surgically useful substances are incorporated into the copolymer.

2. Vascular graft according to claim 1, wherein the one or more medico surgically useful substances are capable of minimizing or preventing platelet adhesion to the surface of vascular grafts.

3. Vascular graft according to claim 1, wherein the one or more medico surgically useful substances are capable of blocking incidents leading to hyperplasia of synthetic vascular grafts.

4. Vascular graft according to claim 1, wherein the one or more medico surgically useful substances are capable of aiding endothelialization of synthetic vascular grafts.

5. Vascular graft according to claim 1, wherein the one or more medico surgically useful substances are capable of preventing smooth muscle cell migration to the lumen of synthetic vascular grafts.

6. Vascular graft according to any of claims 1-5, wherein triethanolamine is used as an initiator in the preparation of the copolymer when the central atom is nitrogen.

7. Vascular graft according to any of claims 1-6, wherein the amorphous component comprises repeat units derived from ε-caprolactone.

8. Vascular graft according to any of claims 1-6, wherein the amorphous component comprises repeat units derived from trimethylene carbonate.

9. Vascular graft according to claim 7, wherein the amorphous component further comprises repeat units derived from trimethylene carbonate.

10. Vascular graft according to any of claims 7-9, wherein the amorphous component further comprises repeat units derived from glycolide.

11. Vascular graft according to any of claims 1-10, wherein the crystallizable component comprises repeat units derived from glycolide.

12. Vascular graft according to claim 11, wherein the crystallizable component further comprises repeat units derived from a second comonomer selected from the group consisting of trimethylene carbonate, ε-caprolactone, ℓ-lactide, ρ-dioxanone, and 1,5 dioxepane-2-one.

## Patentansprüche

1. Gefäßprothese, umfassend ein absorbierbares, kristallines, monozentrisches, polyaxiales Copolymer, umfassend:
ein Zentralatom, ausgewählt aus der Gruppe bestehend aus Kohlenstoff und Stickstoff, und mindestens drei Achsen, die am Zentralatom beginnen und von diesem nach außen verlaufen, wobei jede Achse Folgendes umfasst:
eine amorphe, flexible Komponente, die dem Zentralatom benachbart ist und bei diesem beginnt, wobei die amorphe Komponente Wiederholungseinheiten umfasst, die von mindestens einem cyclischen Monomer stammen, ausgewählt aus der Gruppe, die im Wesentlichen aus Carbonaten und Lactonen besteht, und
mindestens 30, vorzugsweise 65 Gew.-% einer steifen, kristallisierbaren Komponente, die sich von der amorphen, flexiblen Komponente nach außen erstreckt, wobei die kristallisierbare Komponente größtenteils aus von Glykolid oder 1-Lactid stammenden Sequenzen hergestellt ist,
wobei Glycerin, Trimethylolethan, Trimethylolpropan oder Pentaerythritol als Initiator zur Herstellung des Copolymers verwendet wird, wenn das Zentralatom Kohlenstoff ist, und wobei vorzugsweise eine oder mehrere medizinisch-chirurgisch geeignete Substanzen in das Copolymer eingebracht werden.

2. Gefäßprothese nach Anspruch 1, wobei die eine oder die mehreren medizinisch-chirurgisch geeigneten Substanzen in der Lage sind, die Plättchenadhäsion an die Oberfläche von Gefäßprothesen zu minimieren oder zu verhindern.

3. Gefäßprothese nach Anspruch 1, wobei die eine oder die mehreren medizinisch-chirurgisch geeigneten Substanzen in der Lage sind, Vorgänge, die zu einer Hyperplasie synthetischer Gefäßprothesen führen, zu blockieren.

4. Gefäßprothese nach Anspruch 1, wobei die eine oder die mehreren medizinisch-chirurgisch geeigneten Substanzen in der Lage sind, zur Endothelialisierung synthetischer Gefäßprothesen beizutragen.

5. Gefäßprothese nach Anspruch 1, wobei die eine oder die mehreren medizinisch-chirurgisch geeigneten Substanzen in der Lage sind, die Wanderung glatter Muskelzellen in das Lumen synthetischer Gefäßprothesen zu verhindern.

6. Gefäßprothese nach einem der Ansprüche 1-5, wobei Triethanolamin als Initiator bei der Herstellung des Copolymers verwendet wird, wenn das Zentralatom Stickstoff ist.

7. Gefäßprothese nach einem der Ansprüche 1-6, wobei die amorphe Komponente Wiederholungseinheiten umfasst, die von ε-Caprolacton stammen.

8. Gefäßprothese nach einem der Ansprüche 1-6, wobei die amorphe Komponente Wiederholungseinheiten umfasst, die von Trimethylencarbonat stammen.

9. Gefäßprothese nach Anspruch 7, wobei die amorphe Komponente ferner Wiederholungseinheiten umfasst, die von Trimethylencarbonat stammen.

10. Gefäßprothese nach einem der Ansprüche 7-9, wobei die amorphe Komponente ferner Wiederholungs-einheiten umfasst, die von Glykolid stammen.

11. Gefäßprothese nach einem der Ansprüche 1-10, wobei die kristallisierbare Komponente Wiederholungseinheiten umfasst, die von Glykolid stammen.

12. Gefäßprothese nach Anspruch 11, wobei die kristallisierbare Komponente ferner Wiederholungseinheiten umfasst, die von einem zweiten Comonomer stammen, das aus der Gruppe ausgewählt ist, die aus Trimethylencarbonat, ε-Caprolacton, 1-Lactid, p-Dioxanon und 1,5-Dioxepan-2-on besteht.

## Revendications

1. Greffe vasculaire comprenant un copolymère absorbable, cristallin, monocentrique, polyaxial comprenant :
un atome central sélectionné parmi le groupe consistant en carbone et azote; et au moins trois axes naissant de et s'étendant vers l'extérieur à partir de l'atome central, chaque axe comprenant:
un composant amorphe, flexible, adjacent à et naissant de l'atome central, le composant amorphe comprenant des unités de répétition provenant d'au moins un monomère cyclique sélectionné parmi le groupe consistant essentiellement en carbonates et en lactones; et
au moins 30 pour cent, de préférence 65 pour cent en poids d'un composant rigide, cristallisable, s'étendant vers l'extérieur à partir du composant amorphe, flexible, le composant cristallisable étant principalement constitué de séquences dérivées de glycolide ou dérivées de L-lactide,
dans laquelle du glycérol, du triméthyloléthane, du triméthylolpropane ou du pentaérythritol est utilisé en tant qu'initiateur dans la préparation du copolymère lorsque l'atome central est du carbone et dans laquelle de préférence une ou plusieurs substances utiles sur le plan médico-chirurgical sont incorporées dans le copolymère.

2. Greffe vasculaire selon la revendication 1, dans laquelle l'une ou les plusieurs substances utiles sur le plan médico-chirurgical sont capables de minimiser ou de prévenir l'adhésion plaquettaire à la surface de greffes vasculaires.

3. Greffe vasculaire selon la revendication 1, dans laquelle l'une ou les plusieurs substances utiles sur le plan médico-chirurgical sont capables de bloquer des incidents menant à l'hyperplasie de greffes vasculaires synthétiques.

4. Greffe vasculaire selon la revendication 1, dans laquelle l'une ou les plusieurs substances utiles sur le plan médico-chirurgical sont capables de favoriser l'endothélialisation de greffes vasculaires synthétiques.

5. Greffe vasculaire selon la revendication 1, dans laquelle l'une ou les plusieurs substances utiles sur le plan médico-chirurgical sont capables de prévenir la migration cellulaire des muscles lisses vers la lumière de greffes vasculaires synthétiques.

6. Greffe vasculaire selon l'une quelconque des revendications 1-5, dans laquelle de la triéthanolamine est utilisée en tant qu'initiateur dans la préparation du copolymère lorsque l'atome central est de l'azote.

7. Greffe vasculaire selon l'une quelconque des revendications 1-6, dans laquelle le composant amorphe comprend des unités de répétition provenant de ε-caprolactone.

8. Greffe vasculaire selon l'une quelconque des revendications 1-6, dans laquelle le composant amorphe comprend des unités de répétition provenant de carbonate de triméthylène.

9. Greffe vasculaire selon la revendication 7, dans laquelle le composant amorphe comprend en outre des unités de répétitions provenant de carbonate de triméthylène.

10. Greffe vasculaire selon l'une quelconque des revendications 7-9, dans laquelle le composant amorphe comprend en outre des unités de répétition provenant de glycolide.

11. Greffe vasculaire selon l'une quelconque des revendications 1-10, dans laquelle le composant cristallisable comprend des unités de répétition provenant de glycolide.

12. Greffe vasculaire selon la revendication 11, dans laquelle le composant cristallisable comprend en outre des unités de répétition provenant d'un deuxième comonomère sélectionné parmi le groupe consistant en carbonate de triméthylène, en ε-caprolactone, en L-lactide, en p-dioxanone et en 1,5-dioxepane-2-one.
